(19) Europäisches Patentamt
European Patent Office
Office européen des brevets

(11) Veröffentlichungsnummer : **0 077 522**
**B1**

(12) # EUROPÄISCHE PATENTSCHRIFT

(45) Veröffentlichungstag der Patentschrift : **18.09.85**

(51) Int. Cl.⁴ : **C 01 B 33/28**, B 01 J 29/28, C 07 C 1/20, C 07 C 11/02

(21) Anmeldenummer : **82109451.3**

(22) Anmeldetag : **13.10.82**

(54) **Titanhaltige Zeolithe und Verfahren zu ihrer Herstellung sowie ihre Verwendung.**

(30) Priorität : **17.10.81 DE 3141283**
**25.05.82 DE 3219495**
**25.08.82 DE 3231469**

(43) Veröffentlichungstag der Anmeldung :
**27.04.83 Patentblatt 83/17**

(45) Bekanntmachung des Hinweises auf die Patenterteilung : **18.09.85 Patentblatt 85/38**

(84) Benannte Vertragsstaaten :
**BE DE FR GB IT NL**

(56) Entgegenhaltungen :
**FR-A- 2 471 950**
**US-A- 3 329 481**

(73) Patentinhaber : **HOECHST AKTIENGESELLSCHAFT**
**Postfach 80 03 20**
**D-6230 Frankfurt am Main 80 (DE)**

(72) Erfinder : **Baltes, Herbert, Dr.**
**Johannesallee 24**
**D-6230 Frankfurt am Main 80 (DE)**
Erfinder : **Litterer, Heinz, Dr.**
**Albert-Schweitzer-Allee 39**
**D-6200 Wiesbaden (DE)**
Erfinder : **Leupold, Ernst Ingo, Dr.**
**Am Zäunefeld 15**
**D-6392 Neu-Anspach (DE)**
Erfinder : **Wunder, Friedrich, Dr.**
**Jahnstrasse 46**
**D-6093 Flörsheim am Main (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 077 522 B1

**Beschreibung**

Zeolithe sind kristalline Aluminosilicate, bei denen durch eine dreidimensionale Verknüpfung von $SiO_4$- und $AlO_4$- Tetraedern regelmäßige Strukturen mit Hohlräumen und Poren entstehen. Im hydratisierten Zustand sind diese Poren und Hohlar"ume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können gegen andere positiv geladene Ionen ausgetauscht werden. Die geschilderten Eigenschaften ermöglichen die Verwendung der Zeolithe als Ionenaustauscher, Absorbentien und Katalysatoren (D. W.Breck : Zeolite Molecular Sieves, 1974).

Zeolithe des X-, Y-, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom pentasil-Typ (z. B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als katalysatoren besteht großes Interesse an neuen Zeolithen mit spezifischen katalytischen Eigenschaften.

Beispielsweise erhält man sehr interessante Zeolithe, wenn man anstelle von Aluminium und/oder Silizium andere Elemente in das Zeolith-Gerüst einbaut. So wurden unter anderem Zeolithe der Pentasil-Reihe bekannt, die Bor (DE-OS-2 746 790), Eisen (DE-OS-2 831 611), Arsen (DE-AS-2 830 830), Antimon (DE-OS 2 830 787), Vanadin (DE-OS-2 831 631) Chrom (DE-OS-2 831 630) oder Gallium (BE-PS-882 484) auf Tetraederplätzen enthalten.

Auch wurden Titanosilicate (US-PS-3 329 481) und Zirkonosilicate (US-PS-3 329 480) mit Zeolithstruktur bekannt.

Gegenstand der Erfindung sind Titanoaluminosilicate mit Pentasil-Struktur der folgen dem Zusammensetzung :

$$SiO_2 : (0{,}001\text{-}0{,}15)Al_2O_3 : (0{,}002\text{-}1{,}0)TiO_2,$$

ausgedrückt in Molverhältwissen der Oxide.

Für den Begriff Pentasile gilt dabei die Definition von Kokotailo und Meier (« Pentasil family of high silica crystalline materials » in Special Publication n° 33 of the Chemical Society, London 1980). Die Pentasil-Familie umfaßt beispielsweise die synthetischen Zeolithe ZSM-5 (US-PS-3 702 886), ZSM-8 (GB-PS 1 334 243), ZSM-11 (US-PS-3 709 979) und ZSM-23 (US-PS-4 076 842).

Von dem Titanosilicat gemäß US-PS-3 329 481 unterscheidet sich das erfindungsgemäße Titanoaluminosilicat sowohl durch die Struktur wie auch durch den Aluminiumgehalt.

Gegenstand der Erfindung sind vor allem Titanoaluminosilicate mit ZSM-5-Struktur, vorzugsweise solche mit folgender Zusammensetzung, ausgedrückt in Molverhältnissen der Oxide :

$$SiO_2 : (0{,}001\text{-}0{,}15)Al_2O_3 : (0{,}002\text{-}1{,}0)TiO_2$$

insbesondere

$$SiO_2 : (0{,}005\text{-}0{,}1)Al_2O_3 : (0{,}01\text{-}0{,}4)TiO_2.$$

Die erfindungsgemäßen Titanoaluminosilicate lassen sich nach den gleichen Methoden und unter Einsatz der gleichen organischen Verbindungen herstellen, wie sie auch für die Synthese des titanfreien Zeolithen ZSM-5 beschrieben wurden, beispielsweise unter Verwendung von

Alkylammoniumverbindungen (US-PS-3 702 886)
Alkylaminen (US-PS-4 151 189)
Alkyldiaminen (DE-OS-2 817 576, DE-OS-2 831 334)
Alkylaminen in Gegenwart von Alkylierungsmitteln (EP-A-11362, DE-AS-2 212 810)
Aminoalkoholen (GB-PS-2 023 562)
Alkoholen (DE-OS-2 935 123, US-PS-4 199 556, US-PS-4 175 114, EP-OS 42 225, DE-OS-2 643 929)
Ethern (EP-A-51 741)

Vorzugsweise verwendet man Alkylammoniumverbindungen, Alkyldiamine, oder Alkylamine in Gegenwart von Alkylierungsmitteln. Unter den Alkylammoniumverbindungen sind besonders bevorzugt die Tetrapropylammoniumverbindungen, beispielsweise das Hydroxid oder eines der Halogenide. Ein besonders geeignetes Alkyldiamin ist Hexamethylendiamin.

Zur Synthese der erfindungsgemäßen Titanoaluminosilicate mischt man eine oder mehrere Verbindungen aus den genannten Klassen mit Titan-, Silizium-, Natrium- und Aluminiumverbindungen sowie Wasser und erhitzt dieses Gemisch in einem geschlossenen Gefäß. Dem Gemisch werden vorzugsweise darüberhinaus vor dem Erhitzen Impfkristalle eines Pentasils zugesetzt. Falls man Tetrapropylammoniumverbindungen verwendet, werden die Ausgangsverbindungen im allgemeinen in folgendem Verhältnis eingesetzt, ausgedrückt in Molverhältnissen der Oxide :

$$SiO_2 : (0{,}01\text{-}0{,}2)Al_2O_3 : (0{,}01\text{-}1{,}0)TiO_2 : (0{,}01\text{-}0{,}5)Na_2O : (0{,}02\text{-}1{,}0)R_2O : (5\text{-}100)H_2O,$$

vorzugsweise im Verhältnis :

$$SiO_2 : (0{,}01\text{-}0{,}1)Al_2O_3 : (0{,}01\text{-}0{,}4)TiO_2 : (0{,}02\text{-}0{,}3)NaO : (0{,}03\text{-}0{,}6)R_2O : (10\text{-}40)H_2O,$$

wobei R gleich Tetrapropylammonium ist.

Als Silizium-, Aluminium-, Titan- bzw. Natrium-Verbindungen können beispielsweise eingesetzt werden :

Kieselsäuregel, Natriumsilicat, Aluminiumhydroxid, Aluminiumsulfat, Natriumaluminat, Aluminiumhalogenide, Aluminiummethahydroxid, Titanhalogenide, Titansulfat, Natriumhydroxid, Natriumsulfat, Natriumhalogenide. Aber auch andere Verbindungen der vier genannten Elemente eignen sich für die Herstellung der erfindungsgemäßen Zeolithe.

Das Gemisch der jeweils gewählten Verbindungen mit Wasser wird im allgemeinen 18 bis 360 Stunden, vorzugsweise 24 bis 240 Stunden lang, auf eine Temperatur zwischen 100 und 200 °C vorzugsweise zwischen 130 und 170 °C, in einem geschlossenen Gefäß erhitzt.

Die gebildeten Zeolithe werden in üblicher Weise, z. B. durch Filtration, isoliert, gewaschen und getrocknet. Sie können nach bekannten Methoden in die katalytisch aktiven Formen überführt werden, z. B. durch kalzinierung und/oder Ionenaustausch (D.W. Breck, Zeolite Molecular Sieves, 1974).

Die erfindungsgemäßen Zeolithe zeichnen sich nach ihrer Überführung in die katalytisch aktive Form insbesondere aus durch eine hohe Selektivität und durch eine geringe Koksabscheidung bei der Umwandlung von Methanol in niedere Olefine. Diese Reaktion führt man beispielsweise bei Temperaturen von 350 bis 430 °C und einem Wasseranteil im Methanol von 0 bis 80 Gew.-% oder mit Rohmethanol durch.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-α-Strahlung verwandt.

## Beispiel 1

1,66 g Natriumaluminat (54 Gew.-% $Al_2O_3$, 41 Gew.-% $Na_2O$) und 2,74 g Natriumhydroxid werden in 20 g 20 Gew.-%iger wäßriger Tetrapropylammoniumhydroxid-Lösung gelöst (Lösung A).

Eine weitere Lösung (Lösung B) wird hergestellt, indem man 62 g 40 Gew.-%iges kolloidales Kieselgel in 230 g 20 Gew.-%iger wäßriger Tetrapropylammoniumhydroxid-Lösung löst und diese Lösung am Rotationsverdampfer auf insgesamt 230 g einengt. Lösung A und B werden miteinander vermischt. Zu dieser Mischung werden unter intensivem Rühren 2,2 g Titantetrachlorid gegeben. Die entstandene Suspension wird homogenisiert und in einem geschlossenen Gefäß 120 h auf 160 °C erhitzt. Das entstandene Produkt wird abfiltriert, mit Wasser gewaschen und bei 120 °C getrocknet. Man erhält 29,7 g erfindungsgemäßes Titanoaluminosilicat.

Die Röntgenbeugungsanalyse zeigt ein gut kristallines Produkt mit ZSM-5-Struktur. Die chemische Analyse des 16 Stunden bei 540 °C kalzinierten Produktes zeigt folgende Zusammensetzung, ausgedrückt in Molverhältnissen der Oxide :

$$SiO_2 : 0{,}047\ TiO_2 : 0{,}023\ Al_2O_3 : 0{,}051\ Na_2O.$$

## Beispiel 2

Das in Beispiel 1 hergestellte Titanoalyminosilicat mit ZSM-5-Struktur (kalzinierte Form) wird mit Ammoniumnitrat-Lösung ausgetauscht, zusammen mit einem Binder (Boehmit) zu Strängen gepreßt (Zeolith-Anteil 65 Gew.-%) und erneut wie in Beispiel 1 kalziniert.

In einen senkrecht angeordneten, elektrisch beheizten Rohrreaktor von 1 m Länge, der mit 250 ml dieses Katalysators gefüllt ist, dosiert man stündlich 520 ml 33 Gew.-%iges wasserhaltiges Methanol bei einer Temperatur von 350 °C und Normaldruck. Das entstehende Reaktionsgemisch wird abgekühlt, und nach Abtrennung der kondensierbaren Anteile wird die gasförmige Phase analysiert.

Die $C_2$-$C_4$-Olefin-Selektivität ist 64 % und die Selektivität zu Kohlenwasserstoffen mit mehr als 4 C-Atomen ist 13 %.

## Vergleichsbeispiel

Die Arbeitsweise ist wie in Beispiel 2, nur daß statt des Titanoaluminosilicats ein handelsüblicher Aluminosilicat-katalysator mit ZSM-5-Struktur eingesetzt wird.

Die $C_2$-$C_4$-Olefin-Selektivität ist 56 % und die Selektivität zu Kohlenwasserstoffen mit mehr als 4 C-Atomen ist 23 %.

**Patentansprüche**

1. Titanoaluminosilicate mit Pentasilstruktur der folgenden Zusammensetzung :

$$SiO_2 : (0{,}001\text{-}0{,}15)Al_2O_3 : (0{,}002\text{-}1{,}0)TiO_2,$$

ausgedrückt in Molverhältnissen der Oxide.

2. Titanoaluminosilicate gemäß Anspruch 1 mit ZSM-5-Struktur.

3. Titanoaluminosilicate nach einem der Ansprüche 1 bis 2, dadurch gekennzeichnet, daß sie Silizium, Aluminium und Titan im folgenden Verhältnis enthalten :

$$SiO_2 : (0{,}005\text{-}0{,}1)Al_2O_3 : (0{,}01\text{-}0{,}4)TiO_2 ;$$

ausgedrückt in Molverhältnissen der Oxide.

4. Verfahren zur Herstellung von Titanoaluminosilicaten nach einem der Ansprüche 1 bis 3, dadurch gekennzeichnet, daß man Titan-, Silizium-, Natrium-, Aluminiumverbindungen und Wasser mit einer oder mehreren organischen Verbindungen aus der Gruppe bestehend aus Alkylammoniumverbindungen, Alkylaminen, Alkyldiaminen, Aminoalkoholen, Alkoholen und Ethern mischt und diese Mischung in einem geschlossenen Gefäß erhitzt.

5. Verfahren nach Anspruch 4, dadurch gekennzeichnet, daß man als organische Verbindungen Alkylammoniumverbindungen einsetzt.

6. Verfahren nach Anspruch 4, dadurch gekennzeichnet, daß man als organische Verbindungen Tetrapropylammoniumverbindungen einsetzt.

7. Verfahren nach Anspruch 4, dadurch gekennzeichnet, daß man als organische Verbindungen Alkylamine in Gegenwart von Alkylierungsmitteln einsetzt.

8. Verfahren nach Anspruch 4, dadurch gekennzeichnet, daß man als organische Verbindungen Alkyldiamine einsetzt.

9. Verfahren nach einem der Ansprüche 4 bis 8, dadurch gekennzeichnet, daß man der Mischung vor dem Erhitzen Impfkristalle eines Pentasils zusetzt.

10. Verfahren zur Herstellung von Titanoaluminosilicaten nach einem der Ansprüche 1 bis 3, dadurch gekennzeichnet, daß man eine Mischung aus Titan-, Silizium-, Natrium-, Tetrapropylammonium-Aluminiumverbindungen und Wasser herstellt, die folgende Zusammensetzung hat, ausgedrückt in Molverhältnissen der Oxide :

$$SiO_2 : (0{,}01\text{-}0{,}2)Al_2O_3 : (0{,}01\text{-}1{,}0)TiO_2 : (0{,}01\text{-}0{,}5)Na_2O : (0{,}02\text{-}1{,}0)R_2O : (5\text{-}100)H_2O,$$

wobei R gleich Tetrapropylammonium ist, und diese Mischung in einem geschlossenen Gefäß erhitzt.

11. Verfahren nach Anspruch 10, dadurch gekennzeichnet, daß die zu erhitzende Mischung folgende Zusammensetzung hat, ausgedrückt in Molverhältnissen der Oxide :

$$SiO_2 : (0{,}01\text{-}0{,}1)Al_2O_3 : (0{,}01\text{-}0{,}4)TiO_2 : (0{,}02\text{-}0{,}3)Na_2O : (0{,}03\text{-}0{,}6)R_2O : (10\text{-}40)H_2O,$$

wobei R gleich Tetrapropylammonium ist.

12. Verwendung von Titanoaluminosilicaten nach einem dere Ansprüche 1 bis 3 als Katalysatoren bei der Herstellung von $C_2$-$C_4$-Olefinen aus Methanol.

**Claims**

1. Titano-aluminosilicates having a pentasil structure and having the following composition :

$$SiO_2 : (0.001\text{-}0.15)Al_2O_3 : (0.002\text{-}1.0)TiO_2,$$

expressed as molar ration of oxides.

2. Titano-aluminosilicates as claimed in Claim 1 having a ZSM-5 structure.

3. Titano-aluminosilicates as claimed in Claims 1 to 2 which contain silicon, aluminum and titanium in the following ratio :

$$SiO_2 : (0.005\text{-}0.1)Al_2O_3 : (0.01\text{-}0.4)TiO_2,$$

expressed as molar ratio of oxides.

4. A process for the manufacture of titano-aluminosilicates as claimed in one of Claims 1 to 3, which comprises mixing titanium, silicon, sodium, aluminum compounds and water with one or more organic compounds from the group consisting of alkylammonium compounds, alkylamines, alkyldiamines, aminoalcohols, alcohols and ethers, and heating this mixture in a closed vessel.

5. The process as claimed in Claim 4, which comprises using alkylammonium compounds as organic compounds.

6. The process as claimed in Claim 4, which comprises using tetrapropylammonium compounds as organic compounds.

7. The process as claimed in Claim 4, which comprises using alkylamines in the presence of alkylating agents as organic compounds.

8. The process as claimed in Claim 4, which comprises using alkyldiamines as organic compounds.

9. The process as claimed in one of Claims 4 to 8, which comprises adding seed crystals of a pentasil to the mixture before heating.

10. A process for the manufacture of titano-aluminosilicates as claimed in one of Claims 1 to 3, which comprises preparing a mixture of titanium, silicon, sodium, tetrapropylammonium, aluminum compounds and water having the following composition, expressed as molar ratio of oxides :

$$SiO_2 : (0.01\text{-}0.2)Al_2O_3 : (0.01\text{-}1.0)TiO_2 : (0.01\text{-}0.5)Na_2O : (0.02\text{-}1.0)R_2O : (5\text{-}100)H_2O,$$

R being tetrapropylammonium, and heating this mixture in a closed vessel.

11. The process as claimed in Claim 10, wherein the mixture to be heated has the following composition, expressed as molar ratio of oxides :

$$SiO_2 : (0.01\text{-}0.1)Al_2O_3 : (0.01\text{-}0.4)TiO_2 : (0.02\text{-}0.3)Na_2O : (0.03\text{-}0.6)R_2O : (10\text{-}40)H_2O,$$

R being tetrapropylammonium.

12. Use of titano-aluminosilicates as claimed in one of Claims 1 to 3 as catalysts for the manufacture of $C_2$-$C_4$ olefins from methanol.

**Revendications**

1. Titano-aluminosilicates qui ont la structure du Pentasil et qui ont la composition suivante (exprimée en rapports molaires des oxydes) :

$$SiO_2 : (0,001\text{-}0,15)Al_2O_3 : (0,002\text{-}1,0)TiO_2.$$

2. Titano-aluminosilicates selon la revendication 1 qui ont la structure de la ZSM-5.

3. Titano-aluminosilicates selon l'une des revendications 1 et 2, caractérisés en ce que le silicium, l'aluminium et le titane y sont contenus dans les rapports suivants :

$$SiO_2 : (0,005\text{-}0,1)AlO_2 : (0,01\text{-}0,4)TiO_2,$$

exprimés en rapports molaires des oxydes.

4. Procédé de préparation de titano-aluminosilicates selon l'une quelconque des revendications 1 à 3, procédé caractérisé en ce qu'on mélange des composés du titane, du silicium, du sodium et de l'aluminium et de l'eau avec un ou plusieurs composés organiques pris dans l'ensemble constitué par les composés d'alkylammoniums, les alkylamines, les alkylène-diamines, les amino-alcools,les alcools et les éthers, et on chauffe ce mélange dans un récipient fermé.

5. Procédé selon la revendication 4 caractérisé en ce qu'on utilise, comme composés organiques, des composés d'alkylammoniums.

6. Procédé selon la revendication 4 caractérisé en ce qu'on utilise, comme composés organiques, des composés de térapropylammonium.

7. Procédé selon la revendication 4 caractérisé en ce qu'on utilise, comme composés organiques, des alkylamines en présence d'agents d'alkylation.

8. Procédé selon la revendication 4 caractérisé en ce qu'on utilise, comme composés organiques des alkylène-diamines.

9. Procédé selon l'une quelconque des revendications 4 à 8, caractérisé en ce qu'on ajoute au mélange, avant le chauffage, des cristaux d'ensemencement d'un Pentasil.

10. Procédé de préparation de titano-aluminosilicates selon l'une quelconque des revendications 1 à 3, procédé caractérisé en ce qu'on prépare un mélange de composés du titane, du silicium, du sodium, de tétrapropylammonium et d'aluminium et d'eau qui a la composition suivante, exprimée en rapports molaires des oxydes :

$$SiO_2 : (0,01\text{-}0,2)Al_2O_3 : (0,01\text{-}1,0)TiO_2 : (0,01\text{-}0,5)Na_2O : (0,02\text{-}1,0)R_2O : (5\text{-}100)H_2O,$$

où R représente un radical tétrapropylammonium, et on chauffe ce mélange dans un récipient fermé.

11. Procédé selon la revendication 10 caractérisé en ce que le mélange à chauffer a la composition suivante, exprimée en rapports molaires des oxydes :

$$SiO_2 : (0,01\text{-}0,1)Al_2O_3 : (0,01\text{-}0,4)TiO_2 : (0,02\text{-}0,3)Na_2O : (0,03\text{-}0,6)R_2O : (10\text{-}40)H_2O,$$

où R représente un radical tétrapropylammonium.

12. Application de titano-aluminosilicates selon l'une quelconque des revendications 1 à 3 comme catalyseurs dans la préparation d'oléfines en $C_2$-$C_4$ à partir du méthanol.